# EUROPEAN PATENT APPLICATION

(11) **EP 4 011 292 A1**
(43) Date of publication of application: **15.06.2022**
(21) Application number: 20213074.6
(22) Date of filing: 10.12.2020
(51) Int. Cl.: A61B 5/145, A61B 5/00

(54) **SYSTEM AND METHOD FOR OPTICAL SWEAT MEASUREMENT**

(71) Applicant: Koninklijke Philips N.V., 5656 AG Eindhoven (NL)
(72) Inventor: DELLIMORE, Kiran Hamilton J., 5656 AE Eindhoven (NL); VAN LIESHOUT, Ron Martinus Laurentius, 5656 AE Eindhoven (NL); JOHNSON, Mark Thomas, 5656 AE Eindhoven (NL); HUIJBREGTS, Laurentia Johanna, 5656 AE Eindhoven (NL); PELSSERS, Eduard Gerard Marie, 5656 AE Eindhoven (NL)
(74) Representative: Philips Intellectual Property & Standards

(57) **Abstract**

A system for optically measuring the sweat gland activity of an individual comprises an optical sensor for measuring a set of optical signals, each signal for a particular skin location and a controller for determining the sweat gland activity. The controller is configured to process the set of optical signals to determine the number of active sweat glands and recently active sweat glands.

## Description

### FIELD OF THE INVENTION

The invention relates to systems and methods for the optical measurement of sweat. Specifically the systems and methods are intended to optically measure the sweat gland activity of an individual.

### BACKGROUND OF THE INVENTION

Patches are ubiquitously used in the monitoring of patients in clinical settings. While they offer many benefits they also have several shortcomings which limit their use. For instance, they require frequent replacement, which is costly as well as time consuming for clinicians. They may also cause skin irritation due to poor material biocompatibility, and they may produce unreliable measurements due to poor patch-to-skin coupling caused by sweating and ablution which can lead to unintended repositioning of the patch (i.e., migration and/or rotation). Therefore, it is of high interest to find an unobtrusive and optical means to obtain the same monitoring information provided by patches, while eliminating many or all of their aforementioned drawbacks.

Despite dramatic advances in optical patient monitoring using cameras which have enabled vital signs such as heart rate (HR), respiration rate (RR) and blood oxygen saturation (SpO₂) to be reliably measured, the optical monitoring of sweat on the skin surface remains a challenge. Sweat is a non-obtrusively accessible bio-fluid containing physiologically rich information, which when combined with vital signs data can provide clinicians with a more complete real-time snapshot of a patient's health status.

Optical monitoring of sweat is challenging in the clinical context for a number of reasons. Factors that complicate the optical monitoring of sweat include the small volumes (~02 nl/min/gland or less) of sweat produced by sedentary patients and the uneven specular and diffuse reflection on the skin surface which varies with skin colour, the amount of facial oil (i.e., lipids), sebum, sweat and cosmetics (e.g., makeup or lotion) found on the surface of the skin, and is wavelength dependent. Further complications arise from the limited access to anatomical locations where sweat is excreted (e.g., for a bed-bound patient typically only the face and forearms may be optically accessible) and the limitations in the number of active sweat glands on the exposed skin surface area. Finally, interference and noise caused by variations in background illumination due to lighting conditions, caregiver motion during routine care, as well as postural and positional changes of the patient also complicate the optical sweat monitoring.

US 2014/275862 A1 discloses a device for optically measuring the activity of sweat glands. This device uses the interaction between sweat and a mixture of starch and iodine to highlight active sweat glands. The device though is neither capable of providing a continuous measurement of the sweat gland activity nor of determining the development of the sweat gland activity over time.

There remains the need for systems and methods that can be used to optically measure and monitor the sweat gland activity of a person a person with a high degree of accuracy.

### SUMMARY OF THE INVENTION

The invention is defined by the independent claims. The dependent claims define advantageous embodiments.

According to examples in accordance with an aspect of the invention, there is provided a system for optically determining the sweat gland activity of an individual, comprising:
an optical sensor for measuring a set of optical signals, each signal for a particular skin location; and
a controller for determining the sweat gland activity, wherein the controller is configured to process the set of optical signals to determine the number of active sweat glands and the number of recently active sweat glands.

By using optical signals directly to determine the number of active sweat glands and recently active sweat glands without resorting to the use of further agents such as mixtures of iodine and starch the system makes it possible to continuously monitor the sweat gland activity of a person. By determining not just the active sweat glands but also taking into account the recently active sweat glands, the system is able to better contextualize later measurements made on a sweat sample.

In one set of examples, the controller may be configured to compare the set of optical signals or a set of values derived from the set of optical signals to at least a first threshold and a second threshold and to determine the number of active sweat glands and recently active sweat glands based on the result of the comparison.

The determination of the number of active and recently active sweat glands based on a comparison to at least two thresholds is a particularly quick and simple way to determine the number of active and recently active sweat glands.

In another set of examples, the controller may be configured to process the set of optical signals or a set of values derived from the set of optical signals over a time period and to determine the number of active sweat glands and recently active sweat glands based on changes in the optical signals or the set of values derived therefrom over the time period.

The determination of the number of active and recently active sweat glands based on the development of the set of optical signals or values derived therefrom over time can provide more accurate information of the sweat gland activity. Furthermore, determining the development of the optical signals or values derived therefrom over time can provide further information on the development of the sweat gland activity.

Furthermore, the optical signal at each skin location may depend on a local skin surface temperature, such that the set of optical signals is indicative of a temperature profile of the skin surface.

Optical signals depending on the local skin surface temperature can be less prone to interference by ambient influences. Accordingly, performing the determination of the active and recently active sweat glands based on a set of optical signals indicative of a temperature profile of the skin surface can improve the reliability of the determination.

In addition, the system may further comprise a light source illuminating the skin surface.

Illuminating the skin surface increases the precision of the determination of the optical signals or values derived therefrom and/or the trend profile of the optical signals or values derived therefrom.

In some instances, the optical sensor may comprise a micro-lens array.

An optical sensor comprising a micro-lens array makes it possible to extract further information during the measurement of the sweat gland activity such as, for example, the size of individual sweat drops. This can e.g. make the measurement of the sweat rate more accurate.

In some instances, the optical sensor may comprise a photophlethysmogram (PPG) sensor to measure a PPG.

By using a PPG sensor in the optical system, it is possible, in addition to the determination of the sweat gland activity, to determine the residence time of sweat and, hence, substances of interest present in the sweat in the sweat gland and in particular in different parts of the sweat gland in addition to the determination of the active sweat glands and recently active sweat glands. This information can provide further insight into the physiological state of an individual.

In some examples the system may further comprise a sensor for a sweat parameter and the controller may be further configured to determine a value of the sweat parameter per sweat gland based on the number of active and recently active sweat glands.

The knowledge of the value of certain sweat parameters per sweat gland can provide valuable physiological information. The determination of sweat parameters, such as the sweat rate, the sweat volume or the concentration of one or more biomarkers excreted with the sweat, though often induces a time delay between the time sweat is excreted and the parameter is determined. In this time delay the number of active sweat glands can change. This change can distort the determination of the value of the sweat parameter per gland if only currently active sweat glands are considered. By taking account of the recently active sweat glands the precision of the determination of the value of a sweat parameter per gland can be further improved. In one example the conversion of the measured biomarker concentration in the sweat to an equivalent value in the blood depends upon the rate of sweat production from an individual gland at the time that the sweat being analysed was secreted from the sweat gland.

The invention further provides a method of optically determining the sweat gland activity of an individual, comprising the steps of:
measuring a set of optical signals, each signal for a particular skin location; and
determining the number of active sweat glands and the number of recently active sweat glands based on the set of optical signals.

This is the method of using the system defined above. By using optical signals directly to determine the number of active sweat glands and recently active sweat glands without resorting to the use of further agents such as mixtures of iodine and starch the system makes it possible to continuously monitor the sweat gland activity of a person. Furthermore, the method makes it possible to better contextualize later measurements made on a sweat sample.

The present method may further comprise comparing the set of optical signals or a set of values derived from the set of optical signals to at least a first threshold and a second threshold and determining the number of active sweat glands and recently active sweat glands based on the result of the comparison.

The method may further comprise processing the set of optical signals or a set of values derived from the set of optical signals over a time period and determining the number of active sweat glands and recently active sweat glands based on changes in the optical signals or the set of values derived therefrom over the time period.

As explained above, this provides more accurate information of the sweat gland activity and can provide further information on the development of the sweat gland activity.

In the method the optical signal at each skin location may depend on a local skin surface temperature, such that the set of optical signals is indicative of a temperature profile of the skin surface.

The method may further comprise a step of determining the size of individual sweat drops excreted by active sweat glands.

Measuring the drop size of individual sweat drops can, for example, be achieved using a micro-lens array. Knowing the size of individual sweat drops improves the accuracy of the sweat rate determination.

The present method may further comprise a step of detecting a sweat parameter and a step of determining a value of the sweat parameter per sweat gland based on the number of active and recently active sweat glands. This may provide further improvement of the precision of the determination of the value of a sweat parameter per gland.

The invention further provides a computer program comprising computer program code which is adapted, when said program is run on a computer, to implement the above method for optically determining the sweat gland activity of an individual.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a first example of a system for optically determining the sweat gland activity according to the invention;
Figure 2 shows a graph plotting the volume of sweat excreted by a sweat gland over time;
Figure 3 shows a graph plotting the skin surface temperature over time for an active sweat gland, a recently active sweat gland and an inactive sweat gland;
Figure 4 shows an IR-image of an area of skin including active and recently active sweat glands;
Figure 5 shows a graph plotting the light intensity over time for an active sweat gland, a recently active sweat gland and an inactive sweat gland;
Figure 6 shows an example of optical sensor for use in system of the invention including a micro-lens array;
Figure 7 shows a table correlating the number of active sweat glands with the sweat rate per gland;
Figure 8 shows a first schematic representation of the effect of the wavelength of light on the penetration depth of the light into skin;
Figure 9 shows a second schematic representation of the effect of the wavelength of light on the penetration depth of the light into skin;
Figure 10 shows a second example of a system for optically determining the sweat gland activity according to the invention;
Figure 11 shows a flow diagram for a first example of a method for optically determining the sweat gland activity according to the invention; and

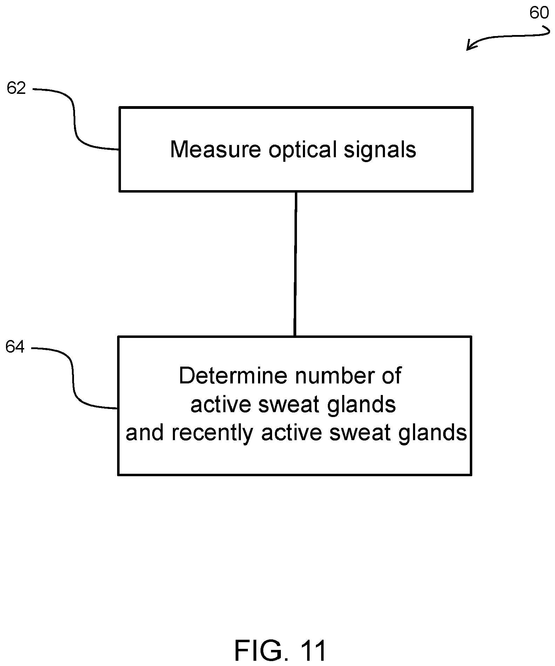

Figure 11 shows a flow diagram for a second example of a method for optically determining the sweat gland activity according to the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides systems and methods for optically determining the sweat gland activity of an individual. The system comprises an optical sensor for measuring a set of optical signals, each signal for a particular skin location and a controller for determining the sweat gland activity. The controller is configured to process the set of optical signals to determine the number of active sweat glands and the number of recently active sweat glands.

For the purpose of this application the term "recently active sweat gland" refers to sweat glands that have stopped to actively excrete sweat but which can still be detected against the background due to the effects of the recently excreted sweat.

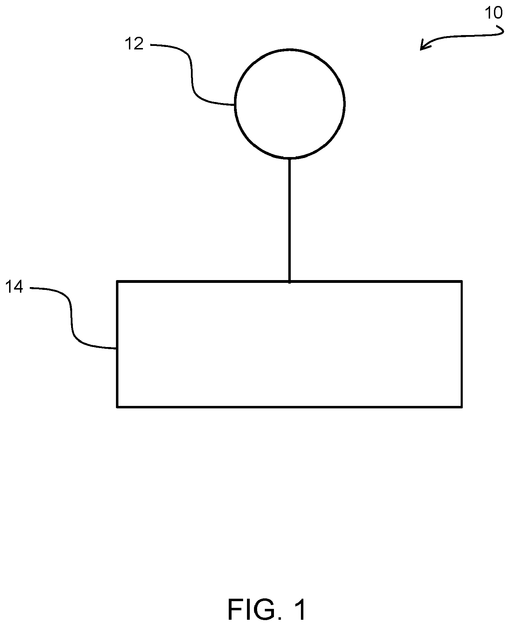

Figure 1 shows an example of a system 10 for optically determining the sweat gland activity of an individual according to the invention. The system 10 comprises an optical sensor 12 for measuring a set of optical signals, each signal for a particular skin location and a controller 14.

The optical sensor 12 can be any optical sensor that is capable of measuring a set of optical signals in a spatially resolved manner. The optical sensor 12 can thereby for example measure electromagnetic radiation at one or more wavelengths. In particular, the optical sensor 12 may be capable of measuring visible light or IR-radiation. The radiation measured by the optical sensor 12 can be reflected by the skin of the individual, for example in the form of visible light. The radiation measured by the optical sensor 12 can also be radiation emitted by the skin of the individual, for example in the form of IR-radiation. Examples for the optical sensor 12 can, for example be, IR-detectors, CCD-arrays, optionally in combination with micro-lens arrays or PPG systems.

The optical sensor may include an optical filter to provide selectivity to a wavelength or wavelength band of interest.

The sensor 12 is in communication with the controller 14. The communication can be wired or wireless, such as a Bluetooth or NFC connection.

The controller 14 receives the set of optical signals from the optical sensor 12. The controller 14 is configured to process the set of optical signals to determine the number of active sweat glands and the number of recently active sweat glands.

The system 10 may further comprise suitable storage, interfaces, output devices or combinations (not depicted here) to store, transmit and/or output the number of active and recently active sweat glands determined by the controller 14.

The controller 14 may be configured to determine the number of sweat glands by processing the set of optical signals or a set of values derived from the set of optical signals over a time period and determining the number of active sweat glands and recently active sweat glands based on changes in the optical signals or the set of values derived therefrom over the time period.

In particular, the processing of the set of optical signals over one time period allows the number of active sweat glands during that time period to be determined as well as the number of sweat glands that were active at a preceding time period, but without the need for analysis of optical signals obtained during that preceding time period.

From this information, a current sweat rate as well as a historic sweat rate may be determined. This information can be used to better contextualize later measurements made on a sweat sample. The knowledge in particular of the historic sweat rate may be used to aid in the calibration of further measurements made on a sweat sample that, due to the nature of the sensors employed, is only possible with a time delay. In particular, it is possible to reliably and accurately determine a sweat rate per gland based on the number of recently active sweat rate in combination with a measurement of the overall sweat volume.

In the above case the skin of an individual may be continuously monitored over time to detect the patterns on an exposed skin surface (e.g., the forehead, cheeks, nose, etc.).

The patterns of the activity of the sweat glands on the skin can be an indication of underlying physiological changes and can provide further useful information. For example a drop in sweat gland activity can be an indication of dehydration. Furthermore, any patterns observed in the sweat gland activity can help to further contextualize any further measurements carried out on a sweat sample.

In some cases, the optical signal at each location depends on a local skin surface temperature, such that the set of optical signals is indicative of a temperature profile of the skin surface. In this case an IR sensitive optical sensor such as a micro-bolometer and optionally an IR light source may be used to monitor thermal patterns on an exposed skin surface. The detected thermal patterns can be analyzed by the image processing unit using frequency decomposition algorithms to estimate the number of active sweat glands, *ρₐₚ* and recently active sweat glands *ρ_{rag}.* This is accomplished by taking advantage of the fact that sweat glands excrete sweat discontinuously.

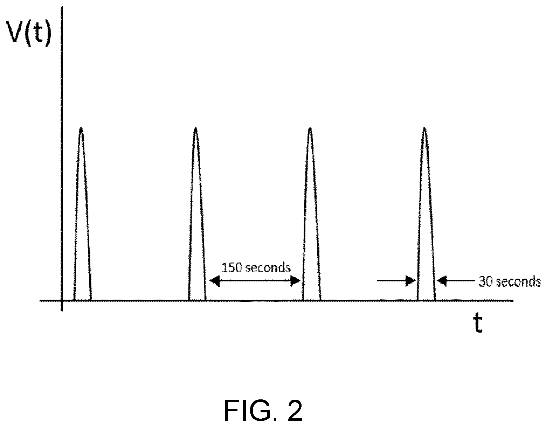

In particular, the sweat glands of the human body do not continuously excrete sweat but do so in intervals followed by a period of inactivity. This excretion behavior is often referred to as pulsation. A sweat gland is on average only actively excreting sweat for 30 seconds within a period of 3 minutes as depicted schematically in Figure 2. Figure 2 plots the volume V(t) of sweat excreted by a sweat gland (arbitrary unit, y-axis) over time (seconds, x-axis) The depiction in Figure 2 is thereby a generalization, and the sweat gland pulsation duty cycle can vary from person to person. Also, depending on the body's thermoregulation, only a fraction of all sweat glands may be active in a given time period, with different sweat glands being active at different times. This pulsation behavior can be detected by optical methods and be used to determine the number of active sweat glands as well as recently active sweat glands. By accounting for gland pulsation it is possible to obtain a more accurate measurement of the number of active glands as well as the recently active sweat glands. This leads to an improved in the precision of optical sweat measurements.

The pulsation of active sweat glands can be detected by the IR optical sensor, by the localized decreasing trend in temperature, due to the cooling effect of the excreted sweat, over the 30 second time period in which the gland is active. This can easily be distinguished from inactive glands, which were either recently active or have been dormant for an extended period. Recently active glands can be detected by a thermal profile which is cooler than the surrounding skin surface. However, since the sweat gland is no longer excreting sweat, the temperature profile will exhibit a localized, slowly rising trend. In contrast, a dormant sweat gland will have the same temperature as the surrounding skin (and therefore is 'invisible' until it becomes active).

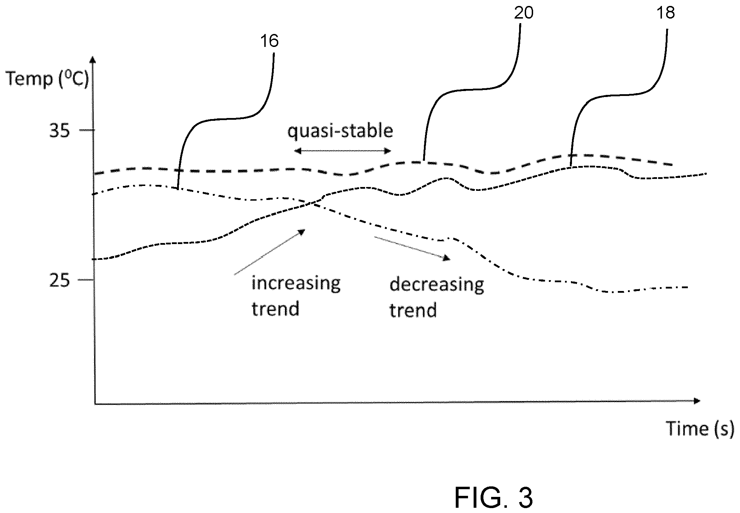

Figure 3 shows a graph plotting the skin surface temperature (°C, y-axis) over time (seconds, x-axis) for an active gland (plot 16), a recently active gland (plot 18) and an inactive gland (plot 20). Plot 16 of Figure 3 thereby clearly shows the decreasing trend in the skin surface temperature for an active sweat gland and plot 18 shows the increasing trend in the skin surface temperature for a recently active sweat gland. Plot 20 shows that the skin temperature for an inactive gland remains quasi stable. This is one approach that permits the quantification of the number of active sweat glands, *ρ_{ag},* and recently active sweat glands, *ρ_{rag}.* The time period over which trends are measured and established are selected to coincide with the duration of the pulsations of the sweat glands, for examples time periods of around 180 seconds.

In another example, the controller 14 may be configured to compare the set of optical signals or a set of values derived from the set of optical signals to at least a first threshold and a second threshold and to determine the number of active sweat glands and recently active sweat glands based on the result of the comparison.

In this case, for example two light intensity cut-off thresholds are defined. The lower light intensity threshold (cut-offl) is indicative of active sweat glands (which appear darker), while the higher threshold (cut-off2) is indicative of inactive sweat glands (which appear brighter, especially if they have not been active for a long time). Light intensities in between the two cut-offs correspond to recently active sweat glands.

A similar approach can also be taken based on the skin surface temperature. The cut-off thresholds can be, for example, based on empirical studies or determined for each individual. It is also possible for the controller 14 to use machine learning or Artificial Intelligence approaches to refine and adjust these cut-off thresholds.

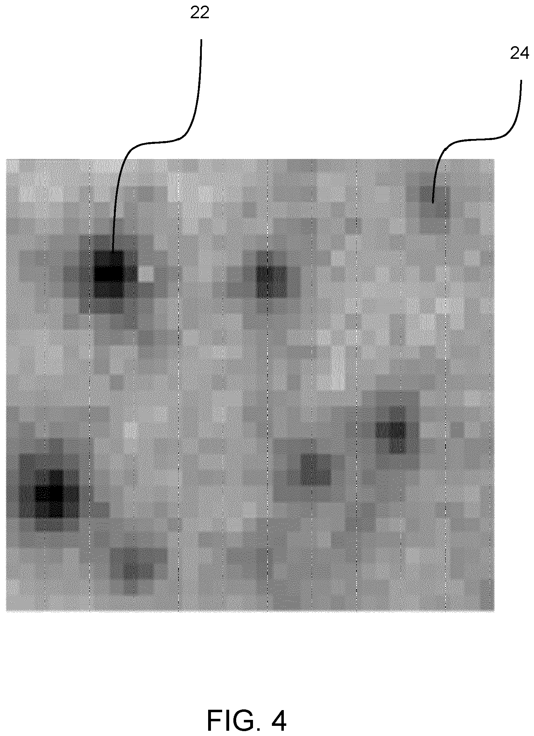

Figure 4 shows an example of a picture of an area of skin comprising both active and recently active sweat glands. In can be clearly seen from this picture that different areas show different light intensities with active sweat glands, such as the one designated with the reference numeral 22, appearing significantly darker than the majority of the skin surface.

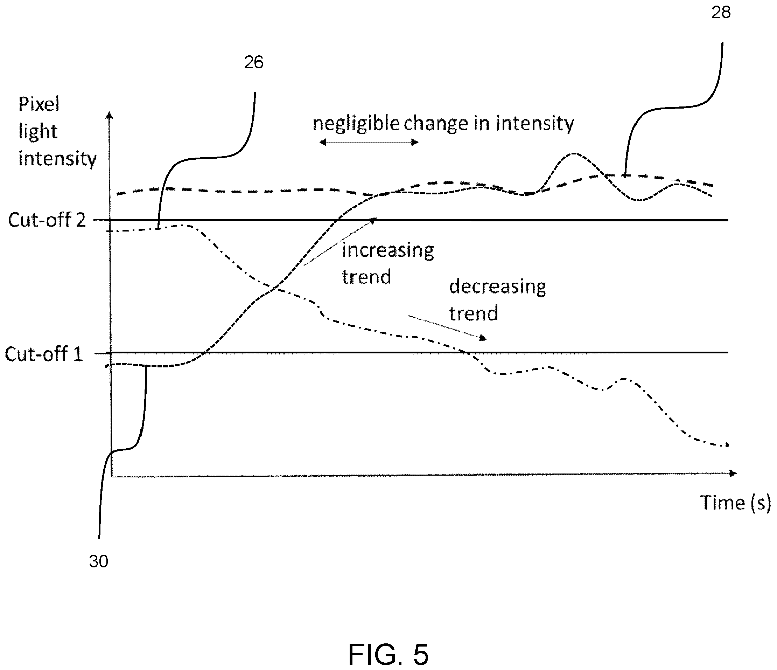

Figure 5 shows a graph plotting the light intensity of a given pixel (arbitrary units, y-axis) over time (seconds, x-axis) for an active gland (plot 26), a recently active gland (plot 30) and an inactive gland (plot 28). In Figure 5, a higher point along the y-axis relates to a higher light intensity. Plot 26 of Figure 5 thereby clearly shows the decreasing trend in the light intensity for an active sweat gland and plot 28 shows the increasing trend in the light intensity for a recently active sweat gland. Plot 28 shows that the light intensity for an inactive gland remains quasi stable.

In the most basic implementation the light intensity is only measured once and compared to the above cut-off values. This will result in a certain amount of uncertainty in the measurement as some active sweat glands early in the excretion cycle will not have dropped below cut-offl and therefore be considered recently active. This uncertainty can be removed by performing the measuring and comparing steps several times over a period of time and, for example, taking the trend in the light intensity into account when determining the number of active sweat glands and recently active sweat glands as described in more detail below.

Referring back to Figure 4, the light intensity of active sweat gland 22 is below cut-offl. Recently active sweat glands, such as the one designated with the reference numeral 24, show a light intensity between that of active sweat glands and that of the background formed by the majority of the skin. The light intensity of recently active sweat gland 24 is above cut-offl and below cut-off2. The light intensity for the majority of the skin surface is above cut-off2.

The above determination can be further refined by also taking into account the change (i.e., trend) in the light intensity over time due to the pulsation of the sweat gland, when determining the activity of the sweat glands. A dip or decreasing trend in brightness is expected for an active sweat gland which is cooling, while a rising trend in brightness is expected for recently active sweat glands. For inactive glands, a negligible change in light intensity is observed. This is illustrated by the graph of Figure 5.

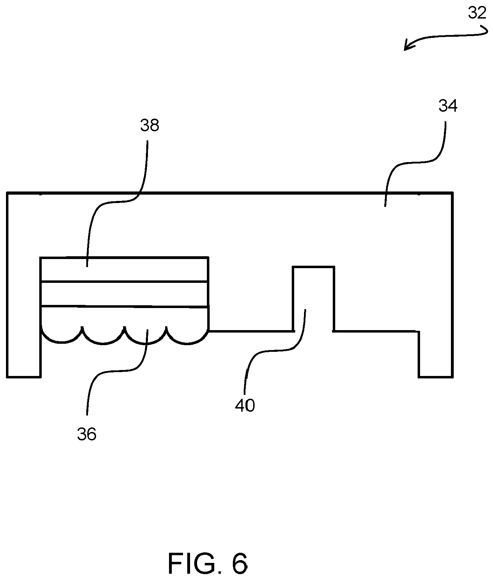

In some instances the optical sensor may comprise a micro-lens array. A device including a micro-lens array that can be employed in the system 10 of the invention is depicted in Figure 6.

The optical sensor 12 forms a part of a sweat sensing device 32. The sweat sensing device 32 comprises a housing 34 which can be attached to the skin of an individual. The device 32 further comprises a micro-lens array 36, which is positioned on the skin-facing side of the device 32 and spaced slightly above the skin at a position where sweat glands are present and also reasonably in the focus of the lenses. The device 32 further comprises a CCD array 38, which is positioned on the side of the micro-lens array 36 away from the skin surface. In use, the skin surface may be illuminated, for example by a lamp from one side or more sides. The light is reflected or scattered from the sweat drop and passes through the micro-lens array 36 which images the drop onto the CCD array 38.

The device 32 further comprises a sensor for a sweat parameter in the form of a microfluidic system 40. The microfluidic system 40 can, for example, be used for biomarker detection or sweat collection for other purposes. Suitable biomarkers may, for example, include sodium or potassium ions or lactate. Any analysis performed by the sensor for a sweat parameter will take a certain amount of time. This amount of time for example includes the amount of time necessary to collect the necessary amount of sweat and the time needed for the analysis. If a calculation of the amount of the value of the sweat parameter per gland is only performed based on the number of sweat glands active at the time the result of the analysis becomes available the result can be distorted. Specifically the conversion of the measured biomarker concentration in the sweat to an equivalent value in the blood depends upon the rate of sweat production from an individual gland at the time that the sweat being analysed was secreted from the sweat gland. For example, the number of active sweat glands can decrease or increase during the time until the analysis is completed, thereby providing a too high or too low value per glad and distorting the analysis of the biomarker blood equivalent. By taking into account the number of recently active sweat glands such distortions can be avoided making the result more reliable.

In addition to the determination of the sweat gland activity, the micro-lens array 36 can be used to determine the size of individual sweat drops. The size of the sweat drop can be measured by analysis of both the size of the image and the intensity of the light incident upon the CCD array 38. The greater the reduction in the intensity of the light, the larger the sweat drop. If the size of the sweat drops and the number of active sweat glands is known, the sweat rate can be determined by counting how many sweat drops are excreted over a given period of time. As this determination is based on the actual size of individual drops, this allows the determination of the sweat rate with a high degree of accuracy.

It has been observed with heat stimulated skin (about 45 degrees Celsius for 3 minutes) that sweat drops occur from eccrine sweat glands within one second and that a drop "disappears" after about an additional second due to evaporation and/or spreading onto the skin. Subsequently, a next drop appears and disappears again. Typically, several of these events occur for a time period of tens of seconds. The latter time period is recognisable as the active period of a gland as depicted schematically in Figure 2. Consequently, by counting the occurrence of these drops, by optical recording, the sweat rate per gland can be derived. Moreover, by recording the occurrence of these drops for a certain prolonged time period and by algorithm analysis, a quantitative distribution of the activity of various glands can be constructed. For instance, when one gland starts to excrete drops the activity over time can yield the sweat rate (by counting drops). It is also possible to record the active period (counting drops) and the inactive period (counting drops) and again the active period and so on. When the glands stop excreting there will be no active period anymore. Since the optical recording is over a skin area, even a distribution of active and non-active glands can be determined.

In general, the resolution of the CCD array 38, for example with a typical pitch of a few microns, far exceeds that of the micro-lens 36 with a typical pitch of 10-100 microns. As a consequence, there are many CCD elements capturing the light from a single micro-lens. Such a situation enables the use of computer optics approaches to further assess details of the drop formation and/or their temperature profile as described above.

It is also possible to consider polarized illumination and/or polarised light detection to increase the quality of the image and the detected signals. Furthermore, an illumination and detection could be carried out from a perpendicular direction by making use of an optionally polarised beam splitter optical arrangement. Such systems are known and, for example, used in optical storage systems.

Furthermore, it is desirable that the detected drops are actually the drops being measured in the device. For this reason, the optical sensor 12 may be integrated with a sweat collection unit of the sweat sensing device 32. In one exemplary embodiment, the surface of the optical window could also be treated with a material with a surface tension gradient running from one side to the other (e.g. right to left) such that the drop is caused to move to one side (e.g. the right) and enter the microfluidic system 40 where it is analysed.

The optical sensor 12 can be integrated into a body worn patch for example in the form of the above micro-lens array 36. The micro lens array 36 is used to pixelate the skin surface, which allows the frequency and/or morphological features within each pixel to be used to determine sweat rate. This is accomplished by utilizing the fact that the presence of sweat will change the pixel intensity at locations where the active sweat glands are excreting sweat via intermittent pulsations. This will allow the number of active sweat glands, *ρ_{ag},* and frequency of sweat gland pulsation to be quantified. Since the patch covers a known area, the number of active glands per unit area, *ρ_{ag}*/*A,* can be computed. The sweat rate is determined from the frequency of sweat gland pulsations, which correspond to the excretion of sweat drops of a particular volume of sweat over a time period, the latter of which can be counted.

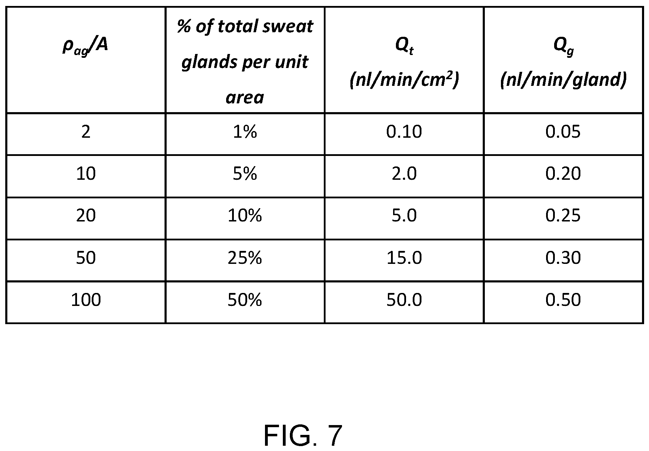

The correlation between pulsation frequency and sweat rate can be obtained in the form of a lookup table. An example of such a lookup table is shown in Figure 7.

Table 7 provides data for the amount of sweat excreted per cm² and minute for given numbers of active sweat glands/unit area. The unit area in this case is 1 cm². The percentage of active sweat glands per unit area is based on assumed number of 200 sweat glands per cm². Qt is the sweat rate per unit area and Qg the corresponding sweat rate per gland.

This can be adapted to account for sweat gland densities in relevant anatomical regions on the body such as the forehead, nose, cheeks and forearms.

In some instances, the optical sensor 12 may comprise a PPG sensor. PPG systems use a light source and a detector at different positions to measure, for example, the pulsation of the blood vessels in the skin. This is used to determine the heart rate and other cardiac-related parameters. The same system may also be used to measure the pulsation of the sweat glands at different depths by adapting the distance between the light source and detector and/or by having multiple wavelengths.

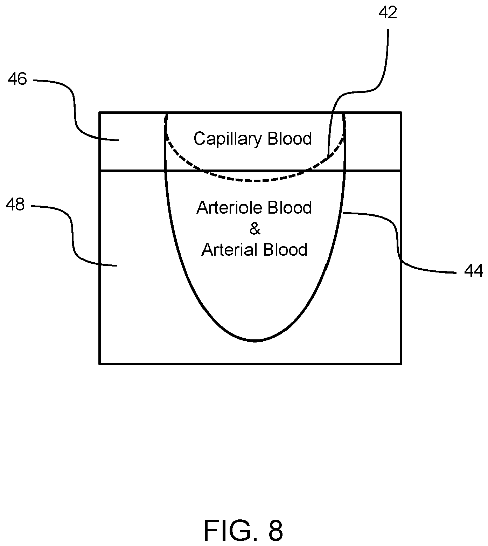

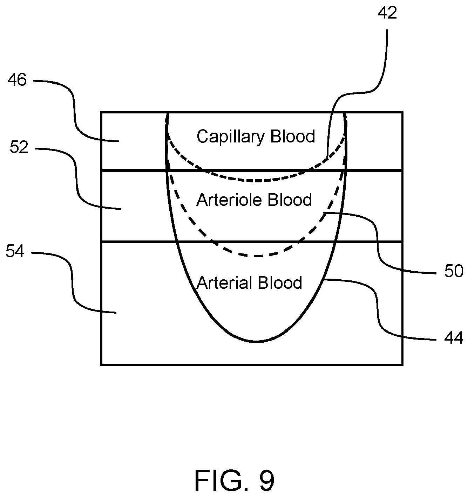

Figures 8 and 9 show schematically the different depths of penetration of light through the skin dependent on the wavelength of the light.

Figure 8 shows a system using light of two different wavelengths with light of a first wavelength being represented by line 42 and light of a second wavelength being represented by line 44. It is clear the two different wavelengths lead to different levels of penetration with the light of the first wavelength mostly being restricted to a first region 46 of the skin comprising the capillary blood and the light of the second wavelength penetration deeper into a second region 48 of the skin comprising the arteriole blood and the arterial blood.

Figure 9 shows that a further differentiation can be achieved by adding light of a third wavelength represented by line 50. This light of a further wavelength now makes it possible to distinguish the above second region 48 of the skin into two sub-regions 52 and 54 comprising the arteriole blood and the arterial blood respectively.

The heart rate and pulsation of the sweat glands are in very different frequency ranges and can therefore be distinguished from each other by applying simple filters, such as digital filters. Accordingly, the heart rate does not interfere with the measurement of the sweat rate using a PPG system.

By using different wavelengths and/or different distances between the illumination source and sensor, the pulsation of the sweat gland at different depths of the gland and duct can be measured. This can be used to determine the 'residence' time in different areas of the gland and duct. This residence time can for example provide further information on the absorption of a molecule of interest into the sweat gland or the speed at which molecules of interest are discharged from the sweat gland. The absorption or reabsorption mechanisms and properties for a molecule of interest, are different for different types of molecules and depend on the location in the duct. By knowing the residence time of the bio-liquid that is measured, a better absorption correction can be made.

The controller 14 may be further configured to detect changes in a physiological state of the individual based on changes in the sweat gland activity.

The immediate observation of a patient is generally an optical one by the caregiver, for example a nurse. It has been reported that among the most prevalent indicators underlying nurse concerns are changes in circulation linked to (new) sweating, as well as skin color and tachycardia, changes in breathing linked to shortness of breath, increased RR and low SpO₂ and changes in body temperature linked to fever. A number of the above vital signs can be measured in an optical and, hence, unobtrusive way.

The system 10 of the invention can optically determine changes in the sweat gland activity and, hence, changes in the sweat rate and can be used to alert a caregiver to a potentially serious deterioration of a patient's condition. As it is well established that nurses' concerns often lead to early recognition of deteriorating patients on general wards in acute care hospitals, the objectification and automatic recognition of the cues used by nurses will lead to an improvement in the early recognition of a deterioration of a patient.

It is thereby also possible to combine the optical sweat measurements of the system 10 of the invention with optical vital signs measurements (e.g., temperature, HR, RR and SpO₂) and other non-vital signs measurements (e.g., skin pallor), to create an integrated system to support early recognition of patient deterioration for example in patients in the general ward or other lower acuity settings. All of these vital and non-vital signs can be measured by the current remote sensing optical technology, for example using systems such as a vital signs camera.

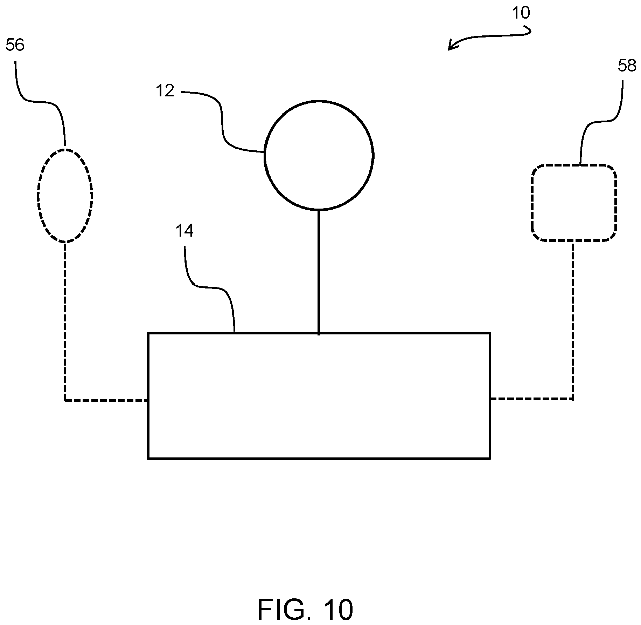

Figure 10 shows a second example of the system 10 of the invention.

In addition to the sensor 12 and the controller 14 the system 10 further comprises a light source 56 to illuminate the skin surface.

Illuminating the skin surface increases the precision of the determination of the optical signals or values derived therefrom and the trend profile of the optical signals or values derived therefrom. The light source can be any light source suitable for use with the optical sensor and can, for example, be a source of visible light or IR radiation.

The system 10 may further comprise a sensor 58 for a sweat parameter. The sensor 58 can be a microfluidic system as described above.

Figure 11 shows a flow diagram for a first example of a method 60 of optically determining the sweat gland activity of an individual.

The method 60 comprises a first step 62 of measuring a set of optical signals, each signal for a particular skin location. This first step can be achieved using any suitable optical sensor, such as for example a micro-lens array combined with an image sensor array or a PPG as described above.

The method 60 further comprises a second step 64 of determining the number of active sweat glands and the number of recently active sweat glands based on the set of optical signals.

As the method 60 of the invention determines not just the active sweat glands but also takes into account the recently active sweat glands, the method 60 is able to measure sweat gland activation as well as the sweat rate of an individual with a higher degree of accuracy.

The method 60 may further comprise processing the set of optical signals of a set or values derived from the set of optical signals over a time period and determining the number of active sweat glands and recently active sweat glands based on changes in the optical signals or the set of values derived therefrom over the time period.

In the method 60, the optical signal at each skin location may depend on a local skin surface temperature, such that the set of optical signals is indicative of a temperature profile of the skin surface.

Optical signals depending on the local skin surface temperature can be less prone to interference by ambient influences. Accordingly, performing the determination of the active and recently active sweat glands based on a set of optical signals indicative of a temperature profile of the skin surface can improve the reliability of the determination.

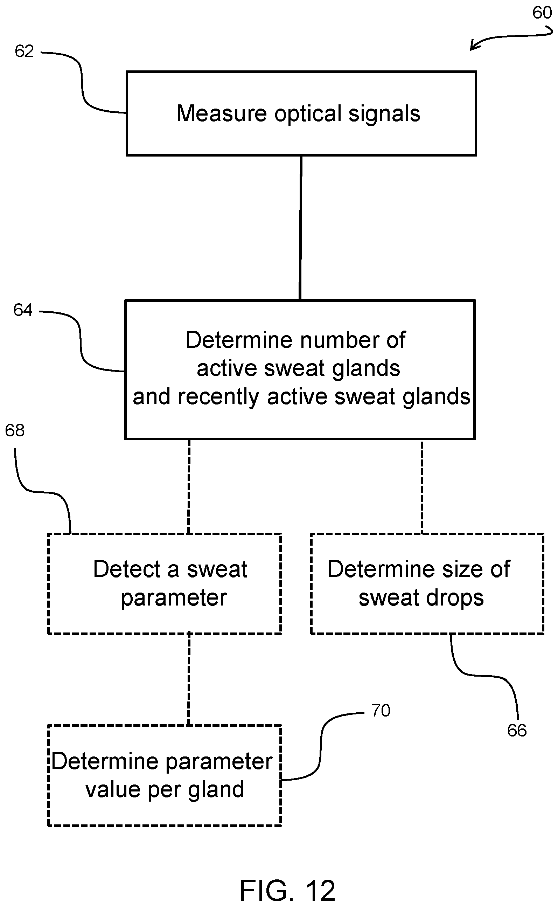

Figure 12 shows a flow diagram for a second example of a method 60 of optically determining the sweat gland activity of an individual.

In addition to the above steps of measuring a set of optical signals and determining the number of active sweat glands and recently active sweat glands, the method 60 further comprises a step 66 of determining the size of individual sweat drops excreted by active sweat glands.

Measuring the drop size of individual sweat drops can, for example, be achieved using a micro-lens array. Knowing the size of individual sweat drops improves the accuracy of the sweat rate determination.

The method 60 further comprises a step 68 of detecting a sweat parameter and a step 70 of determining a value of the sweat parameter per sweat gland based on the number of active and recently active sweat glands.

Determining the value of a sweat parameter taking into account the recently active sweat glands as well as the active sweat glands can provide further improvement of the precision of the determination of the value of a sweat parameter per gland.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for optically determining the sweat gland activity of an individual, comprising:
an optical sensor (12) for measuring a set of optical signals, each signal for a particular skin location; and
a controller (14) for determining the sweat gland activity, wherein the controller (14) is configured to process the set of optical signals to determine the number of active sweat glands and the number of recently active sweat glands.

2. The system of claim 1, wherein the controller (14) is configured to compare the set of optical signals or a set of values derived from the set of optical signals to at least a first threshold, Cut-off1, and a second threshold, Cut-off2, and to determine the number of active sweat glands and recently active sweat glands based on the result of the comparison.

3. The system of claim 1 or claim 2, wherein the controller (14) is configured to process the set of optical signals or a set of values derived from the set of optical signals over a time period and to determine the number of active sweat glands and recently active sweat glands based on changes in the optical signals or the set of values derived therefrom over the time period.

4. The system of any of claims 1 to 3, wherein the optical signal at each skin location depends on a local skin surface temperature, such that the set of optical signals is indicative of a temperature profile of the skin surface.

5. The system of any of claims 1 to 4, further comprising a light source (56) illuminating the skin surface.

6. The system of any of claim 1 to 5, wherein the optical sensor (12) comprises a micro-lens array (36).

7. The system of any of claims 1 to 5, wherein the optical sensor (12) comprises a photophlethysmogram sensor.

8. The system of any of claim 1 to 7, further comprising a sensor (58) for a sweat parameter, wherein the controller (14) is further configured to determine a value of the sweat parameter per sweat gland based on the number of active and recently active sweat glands.

9. A method of optically determining the sweat gland activity of an individual, comprising the steps of:
(62) measuring a set of optical signals, each signal for a particular skin location,; and
(64) determining the number of active sweat glands and the number of recently active sweat glands based on the set of optical signals.

10. The method of claim 9, further comprising comparing the set of optical signals or a set of values derived from the set of optical signals to at least a first threshold and a second threshold and determining the number active sweat glands and recently active sweat glands based on the result of the comparison.

11. The method of claim 9 or claim 10, further comprising processing the set of optical signals of a set or values derived from the set of optical signals over a time period and determining the number of active sweat glands and recently active sweat glands based on changes in the optical signals or the set of values derived therefrom over the time period.

12. The method of any of claims 9 to 11, wherein the optical signal at each skin location depends on a local skin surface temperature, such that the set of optical signals is indicative of a temperature profile of the skin surface.

13. The method of any of claims 9 to 12, further comprising a step (66) of determining the size of individual sweat drops excreted by active sweat glands.

14. The method of any of claim 9 to 13, further comprising a step (68) of detecting a sweat parameter and a step (70) of determining a value of the sweat parameter per sweat gland based on the number of active and recently active sweat glands.

15. A computer program comprising computer program code which is adapted, when said program is run on a computer, to implement the method (62) of any one of claims 9 to 14.
